# EUROPEAN PATENT APPLICATION

(11) **EP 1 439 392 A2**
(43) Date of publication of application: **21.07.2004**
(21) Application number: 04250167.6
(22) Date of filing: 14.01.2004
(51) Int. Cl.: G01N 33/553, C12Q 1/68

(54) **Biosensor systems and methods for determining the presence of biomolecules**

(30) Priority: 15.01.2003 US 342561
(71) Applicant: Agilent Technologies, Inc., Palo Alto, CA 94306 (US)
(72) Inventor: Ihoaka, Seiji, Campbell, CA 95008 (US); Roitman, Daniel B., Menlo Park, CA 94025 (US); Killeen, Kevin Patrick, Palo Alto, CA 94303 (US); Moon, Ronald Leslie, Atherton, CA 94027 (US)
(74) Representative: Tollett, Ian

(57) **Abstract**

Systems and method for determining the presence of biomolecule are disclosed. Briefly described, a representative biosensor system 100 includes a target complex 150 disposed on a conductive substrate 105. The target complex 150 includes a target biomolecule 115 and a target nanoparticle 125. The target nanoparticle 125 is disposed on the target biomolecule 115. In addition, the biosensor includes an image nanoparticle 135 disposed on the target nanoparticle 125. The image nanoparticle 135 is electrochemically deposited on the target nanoparticle 125. The biosensor system also includes a detector capable of determining the presence of the target biomolecule 115 by detecting the image nanoparticle 135.

## Description

The present invention relates to a method and a biosensor system for determining the presence of biomolecules.

In general, a biosensor is capable of identifying biomolecules such as polynucleotides and polypeptides. One method includes introducing a biomolecule sample containing target biomolecules to a substrate having probes deposited thereon. The probe can include various biomolecules that can interact with target biomolecules, while the substrate can be a solid surface such as silica, surface-derivatized glass, polypropylene, and activated polyacrylamide. The target biomolecules can then interact (*e.g.*, bond or hybridize) with one or more of the probes. Subsequently, the interaction can be analyzed based on the presence and location of the target biomolecule-probe interaction, which can be determined by one or more detection techniques such as optical, radiochemical, and electrochemical techniques.

Another method includes introducing a substrate having target biomolecules disposed thereon to a solution that can include complimentary polynucleotide or polypeptide probe samples derived from biomolecules that have been tagged with fluorescent dyes. The probe material interacts selectively with target biomolecues only where complimentary bonding sites occur. In other words, probe biomolecules with similar chemical characteristics (*e.g.*, nucleotide sequence or amino acid sequence) to the target biomolecule interact with the target molecules, while dissimilar probe biomolecules do not significantly interact with the target biomolecules. Thereafter, the presence and quantity of bound probe biomolecules can be detected and analyzed in a manner similar to the detection techniques discussed above.

As stated above, biomolecules can be detected using a variety of detection techniques. However, many of these techniques have disadvantages such as low detection limits, variation in reproducibility of results, and problems with specificity of intercalations. Thus, there is a need in the industry for a biomolecule detection technique that overcomes at least these disadvantages.

Methods and systems for determining the presence of biomolecules are provided. Briefly described, one exemplary system, among others, includes a first target complex disposed on a first conductive substrate. The first target complex includes a first target biomolecule and a first target nanoparticle. The first nanoparticle is disposed on the first biomolecule. In addition, the biosensor includes a first image nanoparticle disposed on the first target nanoparticle. The first image nanoparticle is electrochemically deposited on the first target nanoparticle. The biosensor system also includes a detector capable of determining the presence of the first biomolecule by detecting the first image nanoparticle.

An exemplary method can be broadly summarized by the following steps: providing a first target biomolecule, a first target nanoparticle, and a first image nanoparticle; forming a first image complex electrochemically on a conductive substrate, the first image complex includeing the first target biomolecule, the first target nanoparticle, and the first image nanoparticle, the first image nanoparticle being disposed on the first target nanoparticle, the first target nanoparticle being disposed on the first target biomolecule, and the first target biomolecule being disposed on the conductive substrate; and detecting the first image nanoparticle, which corresponds to the first target biomolecule.

Aspects of a number of preferred embodiments of the present invention can be better understood with reference to the following drawings. The components in the drawings are not necessarily to scale, emphasis instead being placed upon clearly illustrating the principles of the present invention. Moreover, in the drawings, like reference numerals designate corresponding parts throughout the several views.
FIG. 1 is a flow diagram illustrating a representative embodiment of a process for determining the presence of a biomolecule.
FIG. 2 is a flow diagram illustrating a representative embodiment of a process for determining the presence of a biomolecule.
FIG. 3 is a flow diagram illustrating a representative flow of an aspect of the process shown in FIG. 2.
FIGS. 4A through 4E are schematic diagrams of a process for determining the presence of a biomolecule.
FIG. 5 is a flow diagram illustrating a representative embodiment of a process for determining the presence of a biomolecule.
FIG. 6 is a flow diagram illustrating a representative flow of an aspect of the process shown in FIG. 5.
FIGS. 7A through 7D are schematic diagrams of another process for determining the presence of a biomolecule.
FIG. 8 is a flow diagram illustrating a representative embodiment of a process for determining the presence of a biomolecule.
FIG. 9 is a flow diagram illustrating a representative flow of an aspect of the process shown in FIG. 8.
FIGS. 10A through 10E are schematic diagrams of another process for determining the presence of a biomolecule.
FIG. 11 is a flow diagram illustrating a representative embodiment of a process for determining the presence of a biomolecule.
FIG. 12 is a flow diagram illustrating a representative flow of an aspect of the process shown in FIG. 11.
FIGS. 13A through 13D are schematic diagrams of another process for determining the presence of a biomolecule.
FIGS. 14A and 14 B illustrate graphs representing results obtained from a process for determining the presence of biomolecules.
FIG. 15 illustrates results obtained from a process for determining the presence ofbiomolecules.

As will be described in detail here, biosensors capable of determining the presence of biomolecules such as, for example, deoxyribouncleic acid (DNA), ribonucleic acid (RNA), nucleotides, oligonucleotides, nucleosides, proteins, peptides, polypeptides, and antibodies, are provided. In particular, several embodiments will be described that involve determining the presence of biomolecules.

In general, determining the presence of a target biomolecule (*i.e.*, the biomolecule of interest) is accomplished by detecting the presence of image nanoparticles. Image nanoparticles can be associated with the target biomolecule. In this regard, a target nanoparticle is deposited selectively on the target biomolecule through specific interaction between target biomolecule and target nanoparticle. The target biomolecule and the target nanoparticle can selectively interact through biological interactions and/or chemical interactions. The biological interactions can include, but are not limited to, bonding or hybridization among one or more biological functional groups located on the target biomolecule and target nanoparticle. In this regard, the target nanoparticles can include one or more biological functional groups that selectively interact with one or more biological functional groups of the target biomolecule. The chemical interaction can include, but is not limited to, bonding among one or more functional groups (*e.g.*, organic and/or inorganic functional groups) located on the target biomolecule and target nanoparticle. Thus, the target nanoparticle and the target biomolecule can selectively interact with one another to form a "target complex."

Then image nanoparticles are electrochemically deposited from a solution of image nanoparticles on the target nanoparticle. Subsequently, image nanoparticles formed on the target nanoparticle can be detected using techniques such as, for example, imaging techniques, electronic measurement techniques, and/or mass measurement techniques. Detection of the image nanoparticles indicates the presence of the target biomolecule.

As indicated above, the image nanoparticles are electrochemically deposited on the target nanoparticle of the target complex. The electrochemical reaction can be facilitated by the target biomolecule being deposited on a conductive substrate. The target biomolecule can be deposited onto the conductive substrate before or after the formation of the target complex. The target biomolecule attaches to the conductive substrate through one or more selective biological interactions with one or more biological probes disposed on the conductive substrate.

The electrochemical deposition of the image nanoparticles on the target nanoparticle of the target complex occurs when a solution of image nanoparticles is introduced to the target complex. Subsequently, an electric potential is applied to the conductive substrate. The potential can be generated by, for example, the following: instruments such as potentiostats or galvanostats, a battery system capable of supplying appropriate voltages, or contacting a foreign conductor having a different work function than the conductive substrate. These methods can catalyze the deposition of the image nanoparticles on the target complex. In particular, the target nanoparticle acts as a nucleation site to catalytically deposit the image nanoparticles on the target complex to form the image complex.

Thereafter, the image nanoparticles deposited on the target complex can be detected using imaging techniques, electronic measurement techniques, and/or mass measurement techniques. Since the interaction between the target nanoparticle and the target biomolecule is selectively controlled, a direct correlation can be made between the detected image nanoparticles deposited on the target nanoparticle and the target biomolecule. Therefore, the target biomolecule can be qualitatively identified and/or quantitatively measured by detection of the image nanoparticles deposited on the target complex.

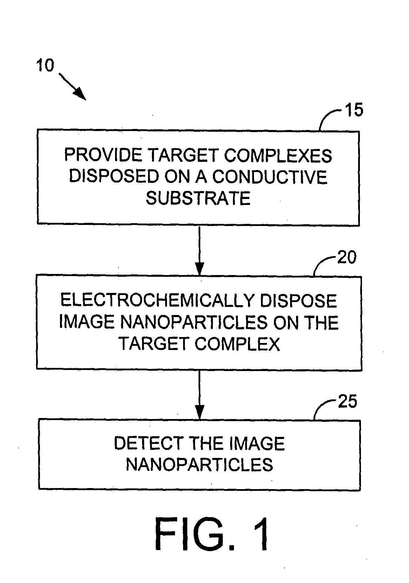

FIG. 1 is a flow diagram illustrating an exemplary process 10 for determining the presence of a target biomolecule. In block 15, target complexes are disposed on a conductive substrate. The target complexes can include, but are not limited to, target biomolecules, target nanoparticles, biomolecule probes, and combinations thereof. Additional details regarding the conductive substrate, the target biomolecule, the target nanoparticle, and the biomolecule probe is discussed below. In block 20, the image nanoparticles are electrochemically deposited on the target complex. In block 25, the image nanoparticles can be detected using imaging techniques, electronic measurement techniques, and mass measurement techniques, which are described in more detail below.

Target nanoparticles are attached (*e.g.*, biologically and/or chemically) to the target biomolecules, which might be attached to the conductive substrate. Alternatively, the target nanoparticles are attached to the target biomolecules, which are attached (*e.g.*, biologically and/or chemically) to the biomolecule probes. In addition, the biomolecule probes are attached to the conductive substrate.

In regard to the detection of the image nanoparticles by imaging techniques, image nanoparticles can be selected so that they have a high optical density. Consequently, the ability to detect the target biomolecule is enhanced by selectively depositing image nanoparticles on the target biomolecules, which have a higher optical density than the image nanoparticles themselves. In other words, the detected image of the image nanoparticles disposed on the target complex corresponds to the biomolecule image of the target nanoparticle. Thus, the biomolecule image of the target biomolecule is amplified by electrochemical deposition of image nanoparticles. Therefore, the target biomolecule can be qualitatively identified and/or quantitatively measured by imaging techniques.

With respect to the detection of image nanoparticles by electrical measuring techniques, the image nanoparticle precursors are selected so that their conversion to image nanoparticles involves an electrochemical reaction, which can be detected by an appropriate instrument such as potentiostat. The deposition of the image nanoparticles facilitates an electrochemical measurement approach that can be used to correlate the deposition of the image nanoparticles to the presence of the target biomolecule.

Another method of using electrical measuring technique includes the measurement of one or more electrical parameters such as resistance, for example. In particular, two conductive substrates having target complexes disposed thereon and located in close proximity to one another can be introduced to a solution of image nanoparticles. As discussed above, the image nanoparticles are deposited on the target nanoparticles of one or both of the conductive substrates. Due to the close proximity of the two conductive substrates, the number of image nanoparticles formed on each target complex increases, which increases the overall size of the mass of image nanoparticles that eventually contact the other conductive substrate. By using image nanoparticles that are conductive, the two conductive substrates are electrically connected through the grown image nanoparticles. Thus, the measurement of electrical properties between the conductive substrates can be performed. If the measurement confirms the existence of the target nanoparticles, then the target biomolecules are present.

For example, the resistance between the two conductive substrates can be measured. Prior to the deposition of the conductive image nanoparticles, the resistance between the two conductive substrates is high since the two conductive substrates are separated from one another. Once the conductive image nanoparticles are formed and merge into the conductive mass of image nanoparticles that encompasses both target complexes, the resistance between the two conductive substrates is reduced. Thus, the resistance can be used as a test parameter for the measurement for the conductive image nanoparticles formed on the target nanoparticle of the target complex. Therefore, the target biomolecule can be qualitatively identified and/or quantitatively measured using electronic measurement techniques.

In regard to the detection of image nanoparticles by mass measurement techniques, the mass of the image nanoparticles can be used to determine the presence of the target biomolecule. In this regard, a conductive substrate having target complexes disposed thereon can be placed upon a mass measuring device, such as but not limited to, a quartz crystal microbalance, to determine the mass of the conductive substrate prior to depositing image nanoparticles thereon. Subsequently, the conductive substrate is introduced to a solution of image nanoparticles. As discussed above, the image nanoparticles are electrochemically formed on the target nanoparticles of the target complex. Thereafter, the mass of the conductive substrate having image nanoparticles deposited on the target complex can be measured using the mass measuring device. Thus, an increase in the mass can be used as a measurement for the deposition of image nanoparticles on the target complex.
Therefore, the target biomolecule can be qualitatively identified and/or quantitatively measured using mass measurement techniques. In contrast, if the conductive substrate did not have target complexes disposed thereon, image nanoparticles would not deposit and consequently there would not be a change in mass of the conductive substrate.

Having described biosensors, *i.e.,* and systems and methods for determining the presence of target biomolecules, in general, four exemplary embodiments will be described.

### Example 1

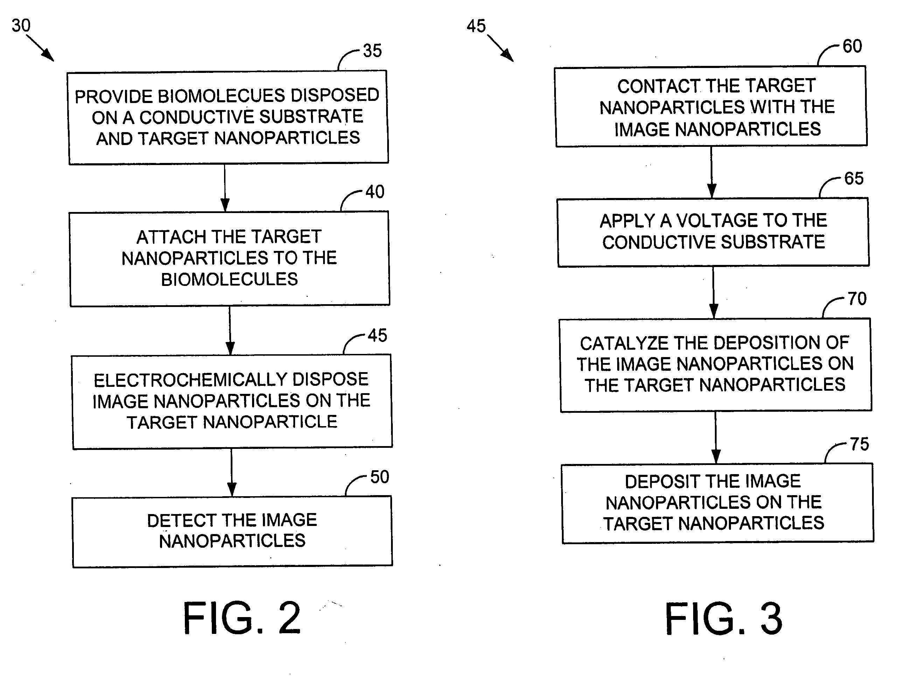

FIG. 2 is a flow diagram illustrating an exemplary process 30 for determining the presence of target biomolecules. In block 35, target biomolecules and target nanoparticles are provided. The target biomolecules are attached directly or indirectly to a conductive substrate via biological and/or chemical interactions. Additional details regarding the target biomolecules, the target nanoparticles, and the conductive substrate are discussed below. In block 40, the target nanoparticles are attached, biologically and/or chemically, to the target biomolecules. In block 45, image nanoparticles are electrochemically deposited on the target nanoparticles. In block 50, the image nanoparticles can be detected using detection techniques such as, but not limited to, imaging techniques, electronic measurement techniques, and mass measurement techniques.

FIG. 3 is a flow diagram illustrating an exemplary aspect of the flow diagram shown in FIG. 2. In particular, block 45 of FIG. 2 illustrates a flow diagram illustrating the steps of electrochemically depositing image nanoparticles on the target nanoparticles. In this regard, image nanoparticles are brought into contact with the target nanoparticles attached to the target biomolecules on the conductive substrate, as shown in block 60. Additional details regarding the image nanoparticles are discussed below. In block 65, a voltage is applied to the conductive substrate. In block 70, the applied voltage catalyzes the deposition of image nanoparticles on the target nanoparticles. As a result, image nanoparticles are deposited on the target nanoparticles, as shown in block 75, through an electrochemical reaction.

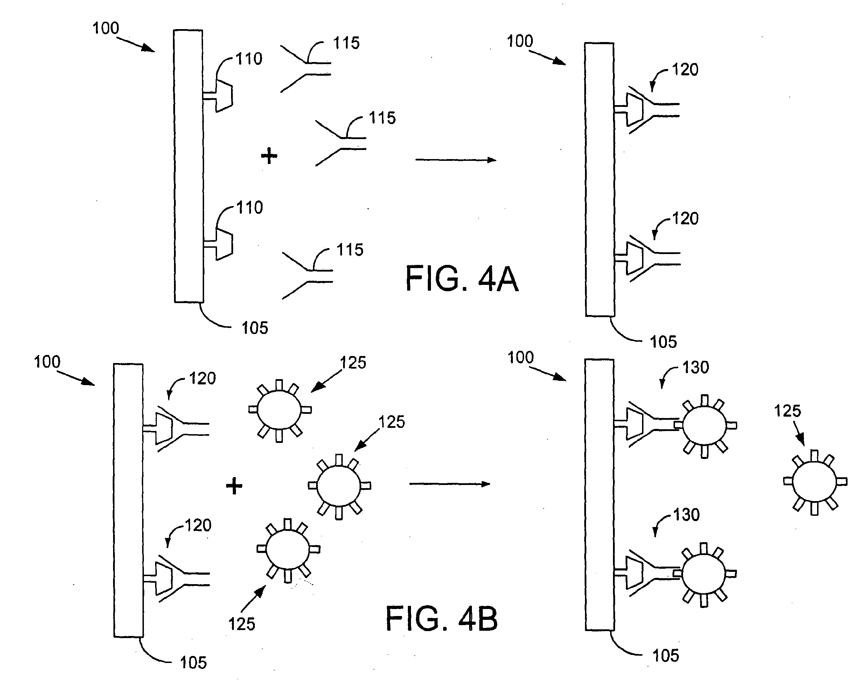

FIGS. 4A through 4E are schematic diagrams collectively illustrating an exemplary process for determining the presence of a target biomolecule for a biosensor system 100. FIG. 4A illustrates the biosensor system 100, where biomolecule probes 110 are disposed on a conductive substrate 105 via biological and/or chemical interactions. In addition, FIG. 4A illustrates introducing target biomolecules 115 to the biomolecule probes 110 to form biomolecule complexes 120.

The biomolecule probe 110 can include biomolecule probes that are capable of attaching to the conductive substrate 105. In addition, the biomolecule probe 110 is capable of selectively attaching to specific target biomolecules 115. For example, the biomolecule probes 110 can include, but are not limited to, deoxyribouncleic acid (DNA), ribonucleic acid (RNA), nucleotides, oligonucleotides, nucleosides, proteins, peptides, polypeptides, and antibodies.

In this embodiment, the biomolecule probes 110 are attached to the conductive substrate 105 prior to being introduced to the target biomolecule 115. In another embodiment, the biomolecule probes 110 can be attached to the target biomolecule 115 prior to being attached to the conductive substrate 105.

The conductive substrate 105 can be any conductive substrate that has an affinity for the biomolecule probes 110. For example, the conductive substrate 105 may be formed of, for example, indium tin oxide (ITO). In addition, the conductive substrate 105 can be designed to have an affinity for different types of biomolecule probes so that multiple biomolecules can be tested at one time. Furthermore, the surface of the conductive substrate 105 can be designed to interact with specific biomolecule probes at predetermined positions (patterned) on the conductive substrate 105. Thus, biosensors can be designed to test for the presence of one or more target biomolecules.

In another embodiment, the conductive substrate 105 can be designed to interact directly with the target biomolecule 115, which would preclude the need for biomolecule probes 110. Furthermore, the surface of the conductive substrate 105 can be designed to interact with one or more specific biomolecules at predetermined positions on the conductive substrate. Thus, biosensors can be designed to test for one or more target biomolecules.

The target biomolecules 115 can include biomolecules such as, for example, deoxyribouncleic acid (DNA), ribonucleic acid (RNA), nucleotides, oligonucleotides, nucleosides, proteins, peptides, polypeptides, and antibodies.

FIG. 4B is a schematic diagram illustrating the introduction of target nanoparticles 125 to the biomolecule complexes 120 to form target complexes 130. As discussed above, target nanoparticles 125 can be disposed on the target biomolecules 115 by interacting selectively (e.g., via biological and/or chemical interactions) with the target biomolecules 115. The target nanoparticles 125 can have one or more moieties and/or one or more functional groups disposed on the target nanoparticle 125 that have an affinity for a particular target biomolecule 115. Therefore, biosensors can be designed to determine the presence of one or more target biomolecules 115 by exposing the target biomolecules 115 to one or more target nanoparticles 125 having known affinities for the particular target biomolecules 115. In particular, the target nanoparticle 125 includes nanoparticles made of materials such as, but not limited to, semiconductive materials (eg., silicon), and metals such as gold, silver, nickel, copper, and platinum.

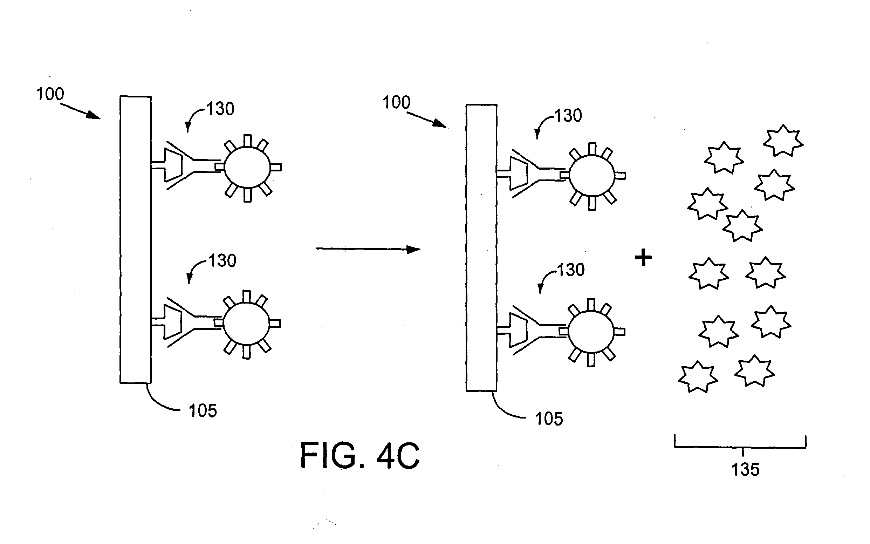

FIG. 4C is a schematic diagram illustrating the introduction of a solution containing image nanoparticles 135 to the target complexes 130. Image nanoparticles 135 can be selected from metals that can be deposited on the target nanoparticle 125. In particular, the image nanoparticle 135 can include, but is not limited to, metal nanoparticles and metal nanoparticle precursors. In particular, the image nanoparticle 135 can include, for example, a silver nanoparticle, a nickel nanoparticle, a copper nanoparticle, a silver nanoparticle precursor, a nickel nanoparticle precursor, and a copper nanoparticle precursor.

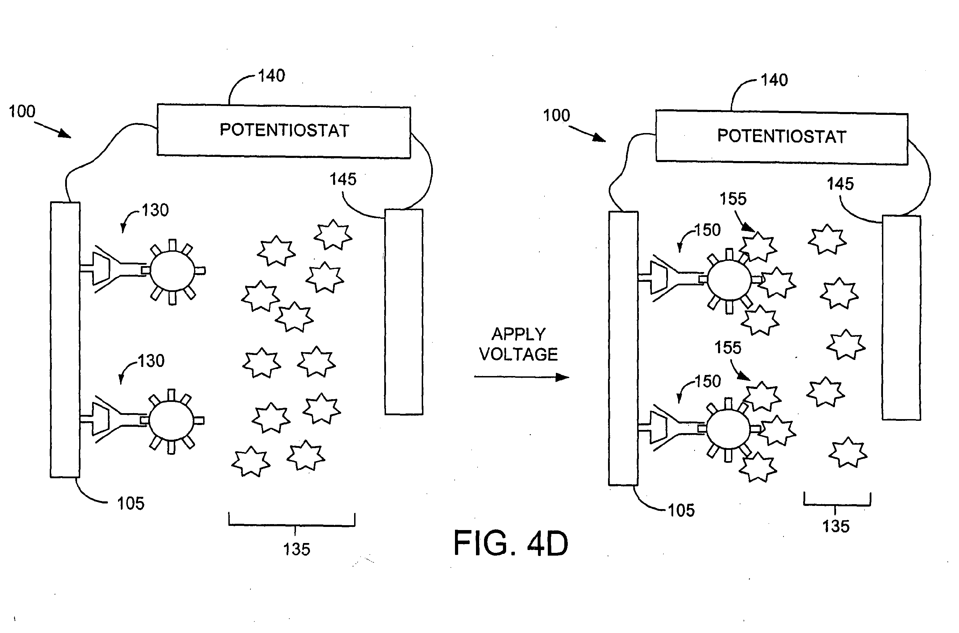

FIG. 4D is a schematic diagram illustrating the application of a voltage to the conductive substrate 105 in the presence of the solution of image nanoparticles 135. The voltage (cathodic) can be applied by a potentiostat 140 having a counter electrode 145 and a reference electrode (not shown), such as silver/silver chloride (Ag/AgCl). The voltage applied to the conductive substrate initiates the deposition of the image nanoparticles 135 on the target complexes 130, thereby forming image complexes 150 on the conductive substrate 105. (General reference, Bard, *et al., Electrochemical Methods: Fundamentals and Application,* 2^{nd} Ed. 2000.)

In particular, the target nanoparticles 125 act as nucleation points for the formation of image nanoparticles by electrochemical reduction on the conductive substrate 105. As a result, the image nanoparticles 135 selectively deposit where the target nanoparticles 125 are disposed on the conductive substrate 105.

Using the image nanoparticles can preserve the original distribution (image) of the target nanoparticles 125 on the surface of the conductive substrate 105. In addition, the image nanoparticles 135 have a high optical density, which can be used to amplify the intensity of the original image of the target biomolecule 115 by forming the image nanoparticles 135 on the target nanoparticles 125.

The potentiostat 140 can control the deposition by adjusting the applied voltage to the conductive substrate 105, In general, the applied voltage can range between about minus1500 millivolts and 1500 millivolts (versus Ag/Agcl reference electrode). However, it should be noted that the applied voltage can vary depending on material for the conductive substrate 105, the target nanoparticle 125, and the image nanoparticle 135.

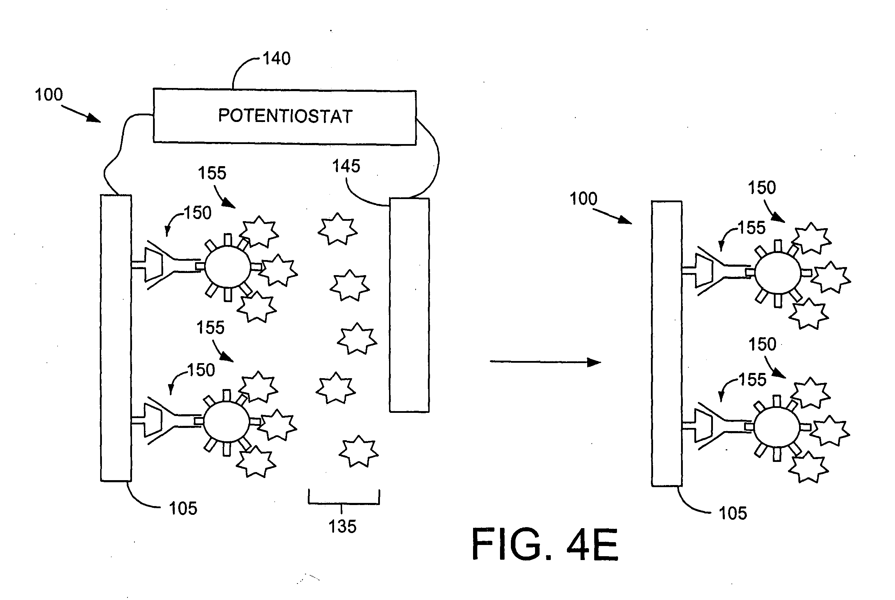

FIG. 4E is a schematic diagram illustrating the separation (e.g., rinsing) of the image nanoparticles 135 and the remaining solution from the image complexes 150 disposed on the conductive substrate 105. Additional preparation and/or treatment of the image complexes 150 can be performed to facilitate their detection.

After sufficient time, the conductive substrate 105 is removed from the vessel and analyzed to determine the degree of deposition of the image nanoparticles 155. The biomolecule image of the conductive substrate 105 having image nanoparticles 155 disposed on the target nanoparticles 125 can be obtained using an imaging device such as, but not limited to, a standard flatbed scanner including Leaf EasyScan^{TM}, or any digital camera, either of which can be controlled using a standard personal computer.

Subsequently, the biomolecule image can be analyzed using ImagePro^{TM} software, which can determine the densitometry of the biomolecule image data. The densitometry data is a profile (i.e., pixel points) of the area of the biomolecule image (the spot) as a function of optical density. Pixel points can be defined as picture elements that define a unit area of the image and corresponds to an optical density. Therefore, a determination can be made whether the image nanoparticles 135 were deposited on the target nanoparticles 125 or not by measuring the optical density of the biomolecule image. Thus, the target biomolecule 115 is present if the biomolecule image reveals that the image nanoparticles 155 were formed on the target nanoparticle 125.

Additional preparation and/or treatment processes of the image complexes 150 can be performed to facilitate the detection of the image complexes 150. For example, the image complexes 150 can be exposed to a chemical and/or an irradiation process that enhances the ability to detect the image complexes 150.

Furthermore, the imaging technique can include radiochemical imaging techniques to compliment the imaging techniques discussed above. In some instances, the image nanoparticles 135 can be viewed with the human eye, thereby facilitating an inexpensive qualitative detection technique. For example, when a biosensor is designed to only deposit image nanoparticles on a specific type of target biomolecule, visual detection of the image nanoparticles confirms the presence of the target biomolecule. Such a detection technique may be useful when other detection techniques are impractical.

The conditions under which the electrochemical deposition of the image nanoparticles is performed can vary depending upon the design of the biosensor 100. In particular, the following variables can be altered: the type and concentration of the image nanoparticles 135, the settings of the potentiostat 140, the pH of the solution, the concentration of acid and/or base to achieve the appropriate pH, the temperature of the solution, the type and concentration of the target nanoparticle 125, the conductive substrate 105, and the type and concentration of the target biomolecule 125.

### Example 2

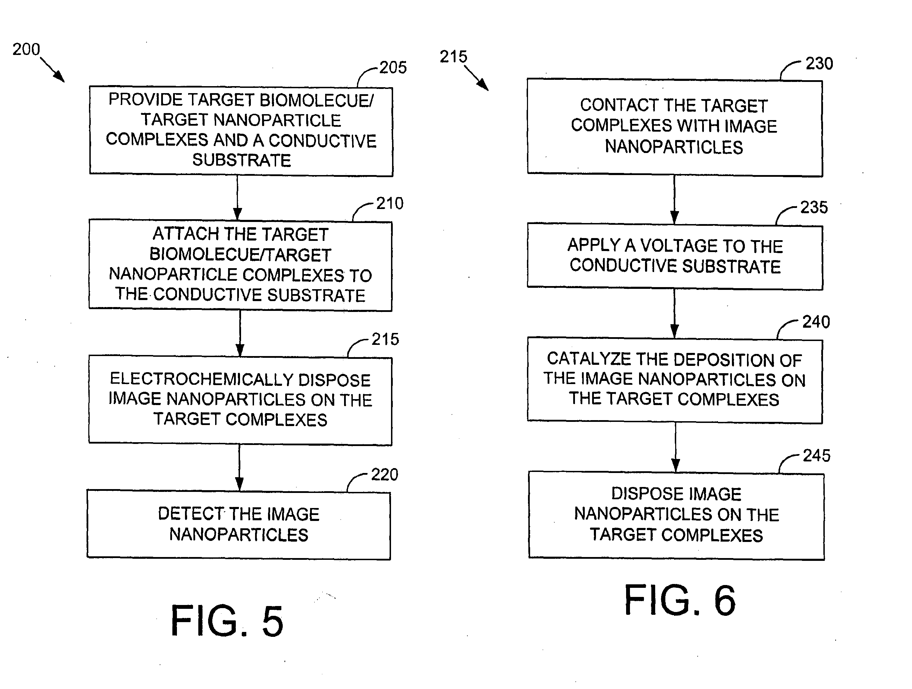

FIG. 5 is a flow diagram illustrating another exemplary process 200 for determining the presence of the target biomolecule. In block 205, target biomolecule/target nanoparticle complexes are provided. The target biomolecule/target nanoparticle complexes include, but are not limited to, target biomolecules and target nanoparticles that have been previously attached to one another. The target nanoparticles are attached to the target biomolecules through biological and/or chemical interactions. Additional details regarding the target biomolecule/target nanoparticle complex are discussed below. In block 210, the target biomolecule/target nanoparticle complexes are attached to a conductive substrate directly or indirectly (e.g., via biological and/or chemical interactions) to form target complexes. Additional details regarding the conductive substrate will be discussed below. In block 215, the image nanoparticles are electrochemically deposited on the target nanoparticles. Additional details regarding the image nanoparticles are discussed below with respect to FIGS. 4A through 4E. In block 220, the image nanoparticles can be detected using techniques such as, for example, imaging techniques, electronic measurement techniques, and mass measurement techniques.

FIG. 6 is a flow diagram illustrating an exemplary aspect of the flow diagram shown in FIG. 5. In particular, block 215 of FIG. 5 illustrates a flow diagram illustrating the steps of electrochemically depositing image nanoparticles on the target nanoparticles. In this regard, the image nanoparticles are brought into contact with the target complexes, as shown in block 230. In block 235, a voltage is applied to the conductive substrate. In block 240, applying the voltage catalyzes the deposition of the image nanoparticles on the target nanoparticle. As a result, the image nanoparticles are deposited on the target nanoparticles, as shown in block 245.

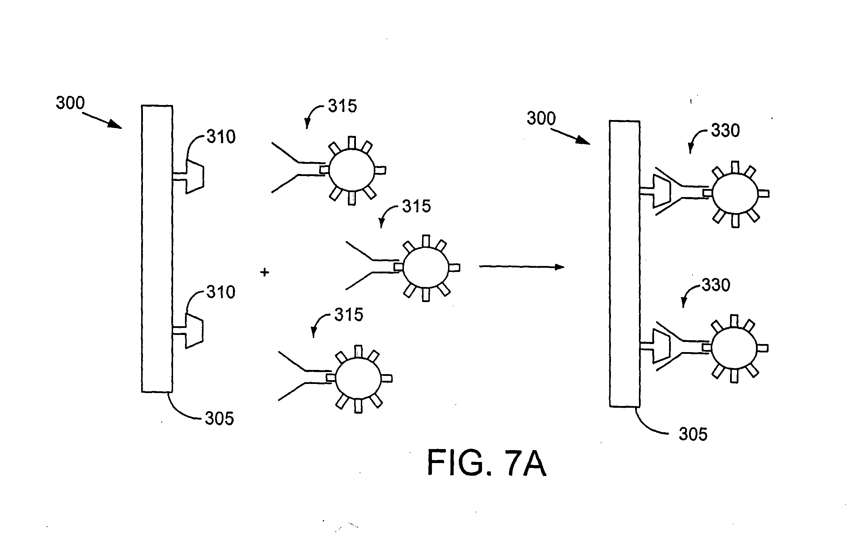

FIGS. 7A through 7D are exemplary schematic diagrams collectively illustrating a process for determining the presence of a target biomolecule for a biosensor system 300. FIG. 7A illustrates the biosensor system 300, where biomolecule probes 310 are disposed on a conductive substrate 305.
In addition, FIG. 7A illustrates the introduction of target biomolecule/target nanoparticle complexes 315 to the biomolecule probes 310 to form target complexes 330. The biomolecule probes 310 are similar to the biomolecule probes 110 discussed in reference to FIGS. 7A through 7E

In this embodiment, the biomolecule probes 310 are attached to the conductive substrate 305 prior to being introduced to the target biomolecule/target nanoparticle complexes 315. In another embodiment, the biomolecule probes 310 can be attached to the target biomolecule/target nanoparticle complexes 315 prior to being attached to the conductive substrate 305. Thus, these configurations as well as other configurations are within the scope of this disclosure.

The conductive substrate 305 is similar to the conductive substrate 105 discussed in reference to FIGS. 4A through 4E. In another embodiment, the conductive substrate 305 can be designed to interact directly with the target biomolecule/target nanoparticle complex 315, which would preclude the need for the biomolecule probe 310. Furthermore, the surface of the conductive substrate 305 can be designed (patterned) to interact with specific biomolecules at predetermined positions on the conductive substrate 305. Thus, biosensors can be designed to determine the presence of one or more target biomolecules.

The target biomolecule can include biomolecules such as, for example, deoxyribouncleic acid (DNA), ribonucleic acid (RNA), nucleotides, oligonucleotides, nucleosides, proteins, peptides, polypeptides, and antibodies.

As discussed above, the target nanoparticles can be disposed on the target biomolecules by biologically and/or chemically interacting selectively with the target biomolecules. The target nanoparticles are similar to the target nanoparticles described in reference to FIGS. 4A through 4E.

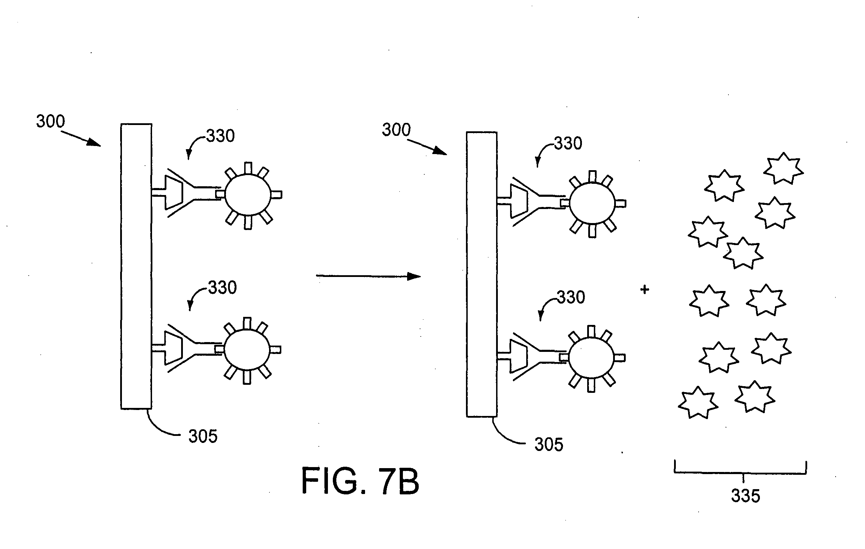

FIG. 7B is a schematic diagram illustrating the introduction of a solution containing image nanoparticles 335 to the target complexes 330. The image nanoparticle 335 is similar to the image nanoparticle discussed in reference to FIGS. 4A through 4E.

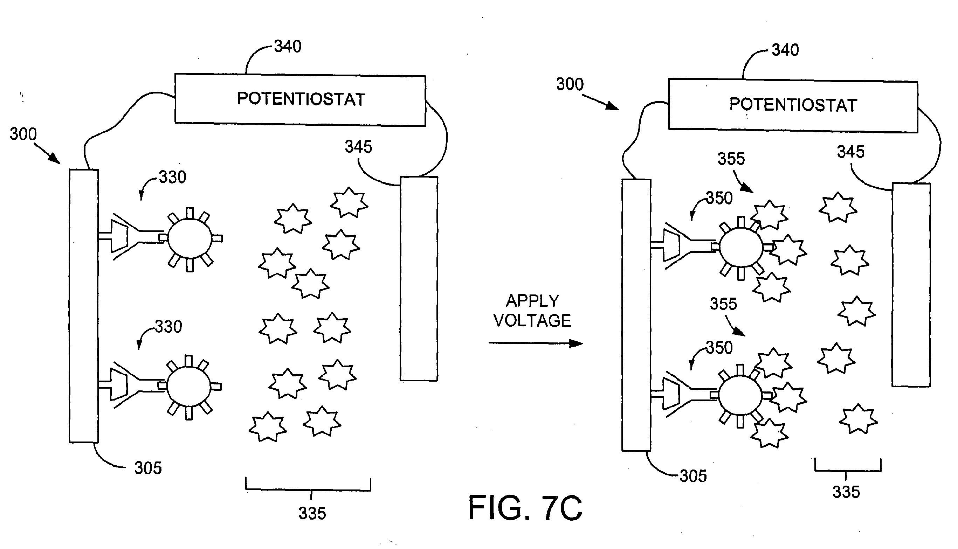

FIG. 7C is a schematic diagram illustrating the application of a voltage to the conductive substrate 305 in the presence of the solution of image nanoparticle precursor 335. The potential can be generated by the techniques discussed in reference to FIGS. 4A through 4E.

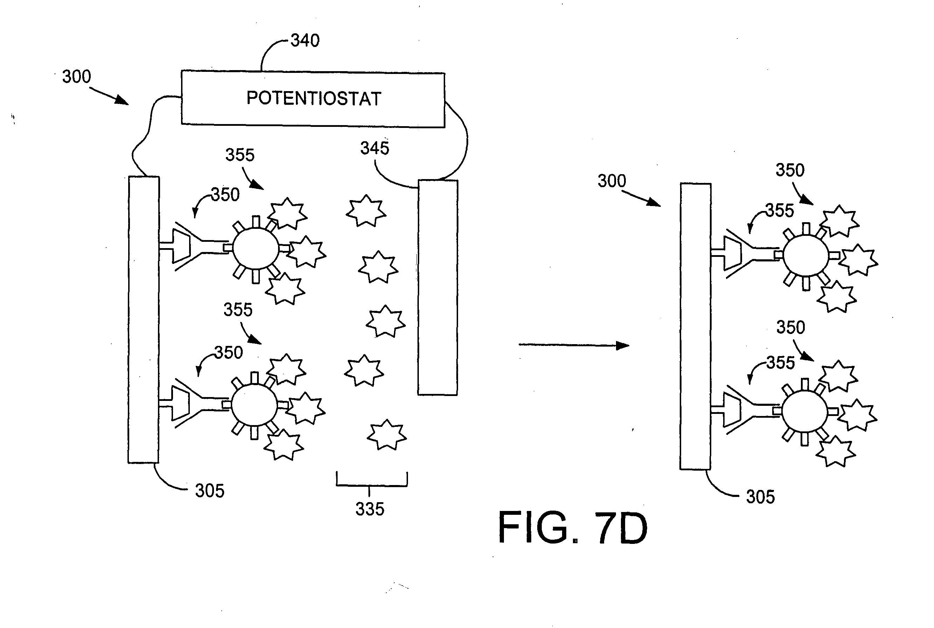

FIG. 7D is a schematic diagram illustrating the separation (e.g., rinsing) of the image nanoparticles 335 and the remaining solution from the image complexes 350 disposed on the conductive substrate 305.

After a few minutes, the conductive substrate 305 can be analyzed to determine the degree of deposition of the image nanoparticles 355 onto the target complexes 330. The biomolecular image of the conductive substrate 305 having image nanoparticles 355 deposited on the target complexes 330 can be obtained using an imaging device and software similar to that discussed in reference to FIGS. 4A through 4D.

As discussed above, additional preparation and/or treatment processes of the image complexes 350 can be performed to facilitate the detection of the biomolecular image.

The conditions under which the electrochemical deposition of the image nanoparticles is performed can vary depending upon the design of the biosensor 300. In particular, the following variables can be altered: the type and concentration of the image nanoparticles 335, the settings of the potentiostat 340, the pH of the solution, the concentration of acid and/or base to achieve the appropriate pH, the temperature of the solution, the type and concentration of the target nanoparticle 325, the conductive substrate 105, and the type and concentration of the target biomolecule 325.

### Example 3

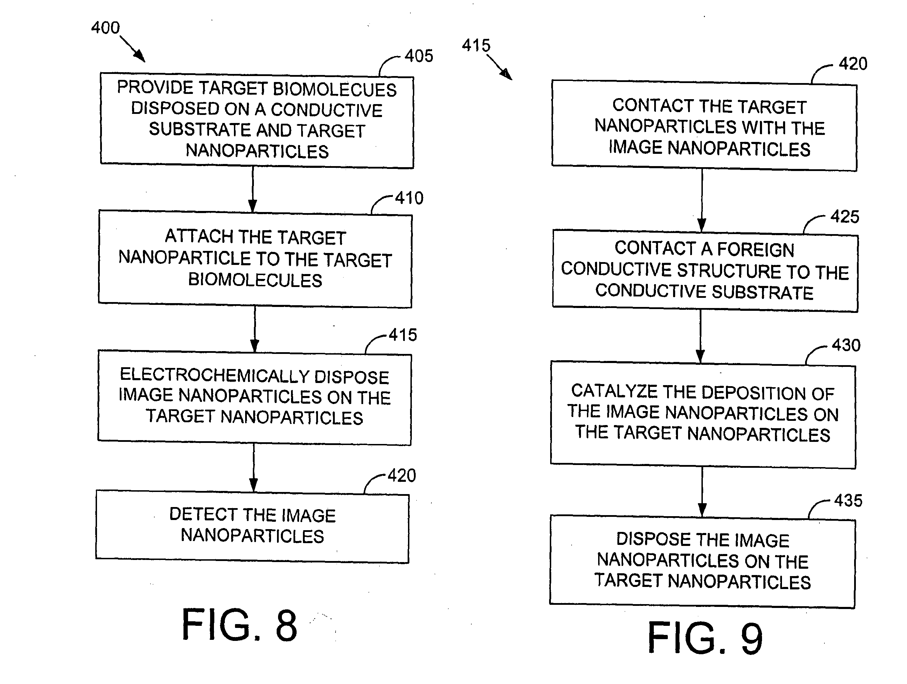

FIG. 8 is a flow diagram illustrating another exemplary process 400 for determining the presence of target biomolecules. In block 405, target biomolecules and target nanoparticles are provided. The target biomolecules are attached to a conductive substrate via biological and/or chemical interactions. Additional details regarding the target biomolecules, the target nanoparticles, and the conductive substrate are discussed below. In block 410, the target nanoparticles are biologically and/or chemically attached to the target biomolecules. In block 415, the image nanoparticles are electrochemically deposited on the target nanoparticles. In block 420, the image nanoparticles can be detected using techniques such as, for example, imaging techniques, electronic measurement techniques, and/or mass measurement techniques.

FIG. 9 is a flow diagram illustrating an exemplary aspect of the flow diagram shown in FIG. 8. In particular, block 415 of FIG. 8 illustrates a flow diagram illustrating the steps of electrochemically depositing image nanoparticles on the target nanoparticles. In this regard, image nanoparticles are introduced to the target nanoparticles attached to the target biomolecules on the conductive substrate, as shown in block 420. Additional details regarding the image nanoparticles are discussed below. In block 425, a foreign conductive structure is contacted (e.g., directly or via a conductive wire) to the conductive substrate. In block 430, contacting the foreign conductive structure to the conductive substrate catalyzes (e.g., using galvanostatic forces) the deposition of the image nanoparticles on the target nanoparticles. As a result, the image nanoparticles are deposited on the target nanoparticles, as shown in block 435.

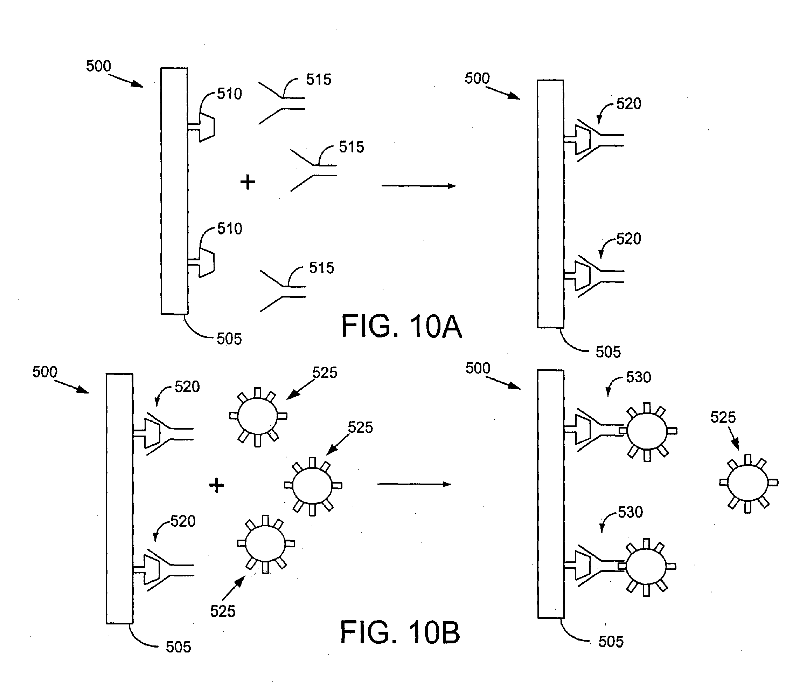

FIGS. 10A through 10E are exemplary schematic diagrams collectively illustrating another process for determining the presence of a target biomolecule for a biosensor system 500. FIG. 10A illustrates the biosensor system 500, where biomolecule probes 510 are deposited on a conductive substrate 505.
In addition, FIG. 10A illustrates the introduction of target biomolecules 515 to the biomolecule probes 510 to form biomolecule complexes 520.

The biomolecule probes 510, the conductive substrate 505, and the target biomolecules 515 are similar to the biomolecule probes 110, the conductive substrate 105, and the target biomolecule 115 discussed in reference to FIGS. 4A through 4E.

FIG. 10B is a schematic diagram illustrating the introduction of the target nanoparticles 525 to the biomolecule complexes 520 to form target complexes 530. The target nanoparticles 125 are similar to the target nanoparticles described in reference to FIGS. 4A through 4E.

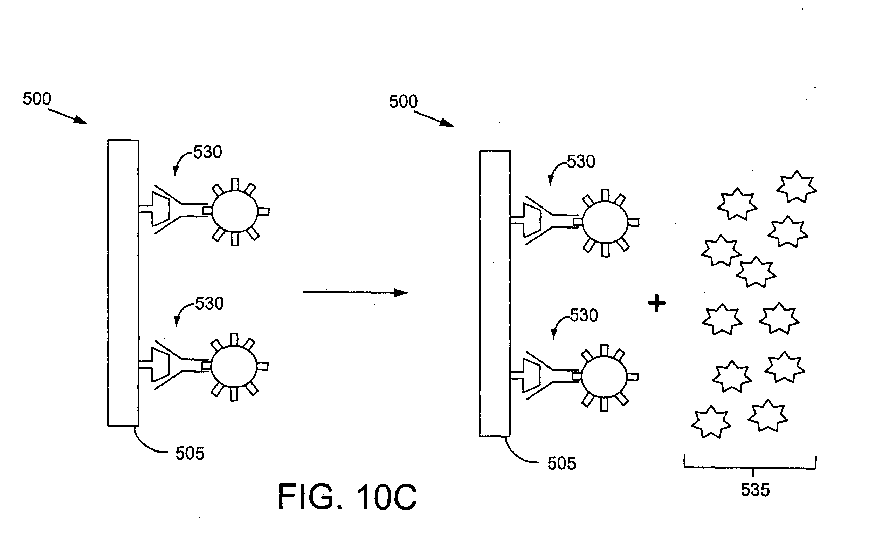

FIG. 10C is a schematic diagram illustrating the introduction of a solution containing image nanoparticles 535 to the target complexes 530. The image nanoparticles 535 are similar to the image nanoparticles described in reference to FIGS. 4A through 4E.

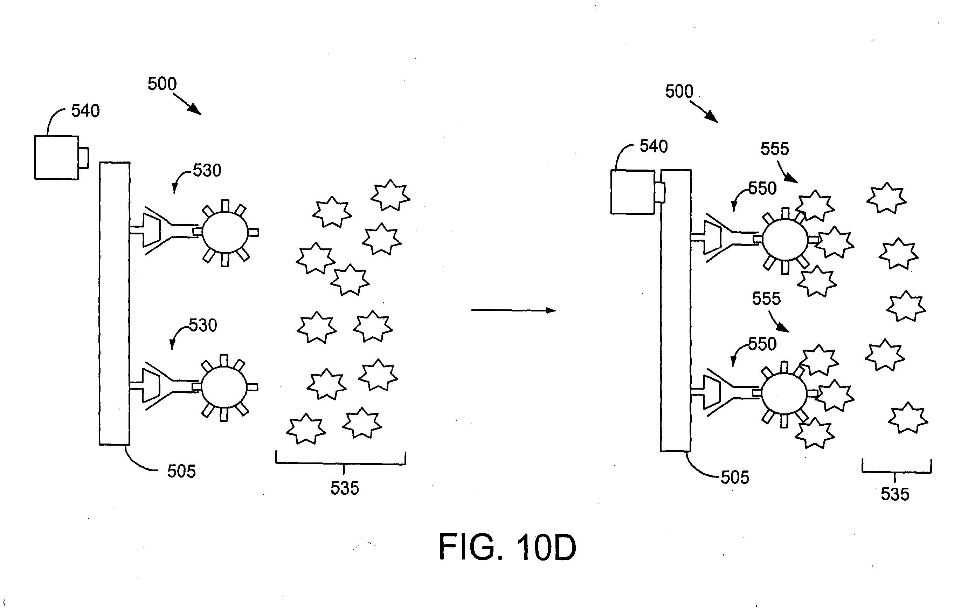

FIG. 10D is a schematic diagram illustrating the introduction of a foreign conductive structure 540 to the conductive substrate 505 in the presence of the solution of image nanoparticles 535. Contacting a portion of the foreign conductive structure 540 initiates the deposition of the image nanoparticles 535 on the target complexes 530, thereby forming image complexes 550 on the conductive substrate 105. In particular, the material for the foreign conductive structure 540 is chosen so that it has a higher tendency to be ionized than the conductive substrate 505. When the foreign conductive structure 540 is contacted to the conductive substrate 505, and both 505 and 540 are immersed in a solution of image nanoparticle 535, a galvanostatic force is created that results in reduction of the surface of the conductive substrate 505, as well as oxidation of the conductive structure 540. The image nanoparticles 535 are reduced on the conductive substrate 505. In addition, the target nanoparticles 525 are made of materials that have a higher work function than the conductive substrate 505. As discussed above, the target nanoparticle 525 is made of metals such as, but not limited to, gold, nickel, copper, or platinum.

When the electron is transferred to the conductive substrate 505 that has the target nanoparticle 525, the extra electron will be concentrated to the surface of the target nanoparticle 525, because the target nanoparticle 525 has a lower energy state than the conductive substrate 505. Therefore, the negative charge is concentrated to the surface of the target nanoparticle 525, which causes localized reduction of the image nanoparticles 535.

When the foreign conductive structure 540 and the conductive substrate 505 are immersed in the solution having the image nanoparticles 535 and are electrically connected, the image nanoparticles 535 are catalytically reduced on the target nanoparticles 525. The target nanoparticles 525 act as nucleation sites for the deposition of the image nanoparticles 535, and as a result, the image nanoparticles 535 selectively form at the location where the target nanoparticles 525 are disposed.

The foreign conductive structure 540 can be made of materials such as, but not limited to, copper, nickel, and iron, their alloys, and combinations thereof. Preferably, the foreign conductive structure 540 is a copper conductive structure.

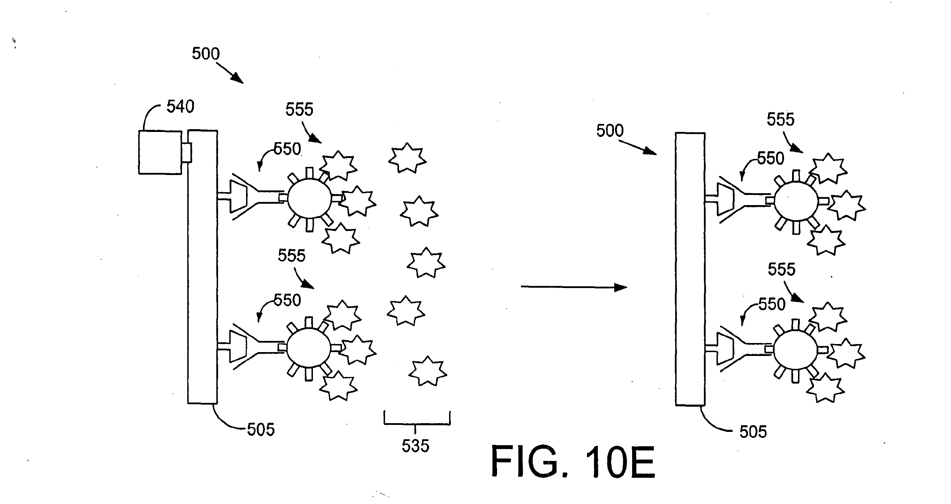

FIG. 10E is a schematic diagram illustrating the removal of the foreign conductive structure 540 from the conductive substrate 505, as well as the separation (e.g., rinsing) of the image nanoparticles 535 and the remaining solution from the image complexes 550 disposed on the conductive substrate 505. Additional preparation and/or treatment of the image complexes 550 can be performed to facilitate their detection.

After sufficient time, the conductive substrate 505 can be analyzed to determine the degree of deposition of the image nanoparticles 555. The biomolecule image of the conductive substrate 505 having image nanoparticles 555 disposed on the target nanoparticles 525 can be obtained using an imaging device and software as described in reference to FIGS. 4A through 4E.

As discussed above, additional preparation and/or treatment processes of the image complexes 550 can be performed to facilitate the detection of the image complexes 550.

The conditions under which the electrochemical deposition of the image nanoparticles is performed can vary depending upon the design of the biosensor 500. In particular, the following variables can be altered: the type and concentration of the image nanoparticles 535, the type of foreign conductive structure 540, the pH of the solution, the concentration of acid and/or base to achieve the appropriate pH, the temperature of the solution, the type and concentration of the target nanoparticle 525, the conductive substrate 505, and the type and concentration of the target biomolecule 525.

### Example 4

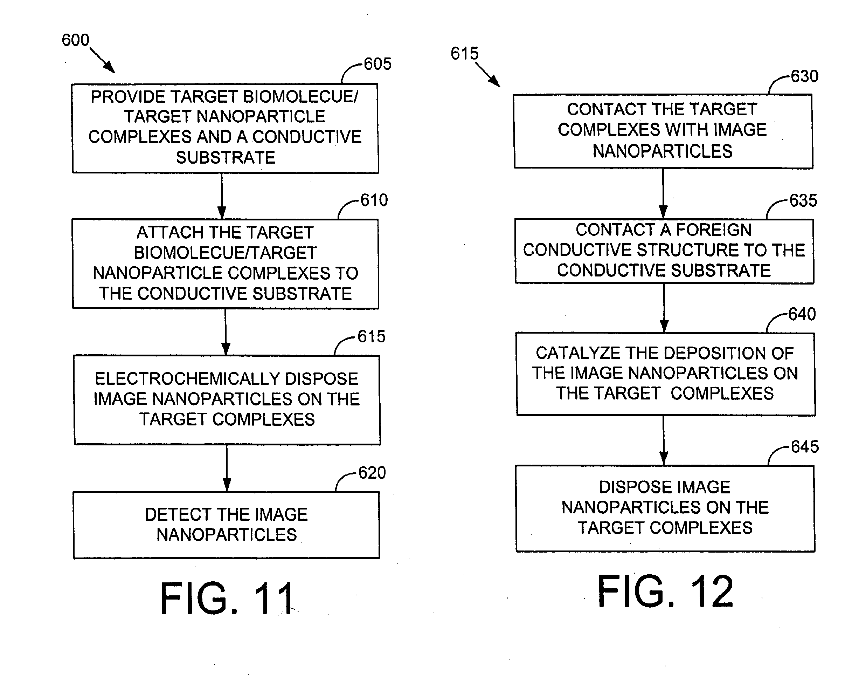

FIG. 11 is a flow diagram illustrating another exemplary process 600 for determining the presence of the target biomolecule. In block 605, target biomolecule/target nanoparticle complexes are provided. The target nanoparticles are attached, biologically and/or chemically, to the target biomolecules. Additional details regarding the target biomolecule/target nanoparticle complexes will be discussed below. In block 610, the target biomolecule/target nanoparticle complexes are attached to a conductive substrate via biological and/or chemical interactions. Additional details regarding the conductive substrate will be discussed below. In block 615, image nanoparticles are electrochemically deposited on the target nanoparticles of the target biomolecule/target nanoparticle complexes to form target complexes. Additional details regarding the image nanoparticles are discussed below. In block 620, the image nanoparticles can be detected using techniques such as, for example, imaging techniques, electronic measurement techniques, and/or mass measurement techniques.

FIG. 12 is a flow diagram illustrating an exemplary aspect of the flow diagram shown in FIG. 11. In particular, block 615 of FIG. 11 illustrates a flow diagram illustrating the steps of electrochemically depositing image nanoparticles on the target biomolecule/target nanoparticle complexes. In this regard, image nanoparticles are introduced to the target biomolecule/target nanoparticle complex on the conductive substrate, as shown in block 630. Additional details regarding the image nanoparticles are discussed below. In block 635, a foreign conductive structure is contacted (*e.g.*, directly or indirectly via a conductive wire) to the conductive substrate. In block 640, contacting the foreign conductive structure to the conductive substrate catalyzes (*e.g.*, using galvanostatic forces) the deposition of the image nanoparticles on the target complexes. As a result, the image nanoparticles are deposited on the target complexes, as shown in block 645.

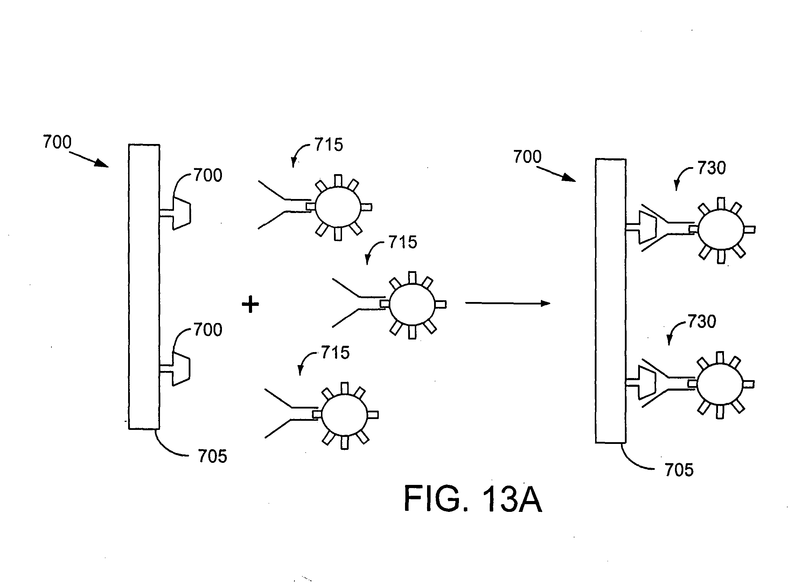

FIGS. 13A through 13E are exemplary schematic diagrams collectively illustrating another process for determining the presence of a target biomolecule for a biosensor system 100. FIG. 13A illustrates the biosensor system 700, where biomolecule probes 710 are disposed on a conductive substrate 705.
In addition, FIG. 13A illustrates introducing the target biomolecule/target nanoparticle complexes 715 to the biomolecule probes 710 to form target complexes 720.

The biomolecule probe 710, the conductive substrate 705, the target biomolecule, and the target nanoparticle, are similar to the biomolecule probes 110, the conductive substrate 105, the target biomolecule 15, and target nanoparticle 125 described in reference to FIGS. 4A through 4E.

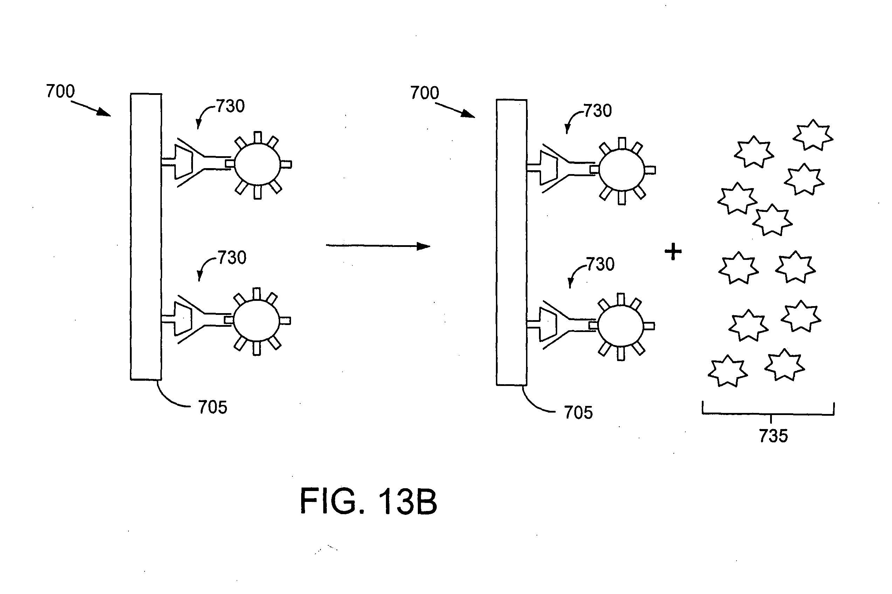

FIG. 13B is a schematic diagram illustrating the introduction of a solution containing image nanoparticles 735 to the target complexes 730. The image nanoparticles 735 are similar to the image nanoparticles 135 described in reference to FIGS. 4A through 4E.

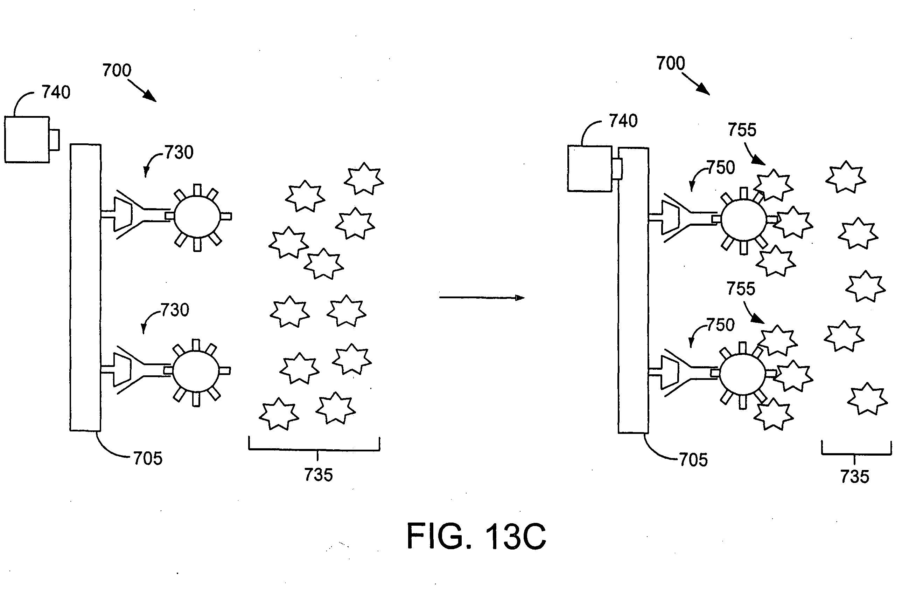

FIG. 13C is a schematic diagram illustrating the introduction of a foreign conductive structure 740 to the conductive substrate 705 in the presence of the solution of image nanoparticles 735. Contacting the foreign conductive structure 740 to the conductive substrate 705 initiates the deposition of the image nanoparticles 735 on the target complexes 730, thereby forming image complexes 750 on the conductive substrate 105. The foreign conductive structure 740 is similar to the foreign conductive structure 540 described in reference to FIGS. 10A through 10E.

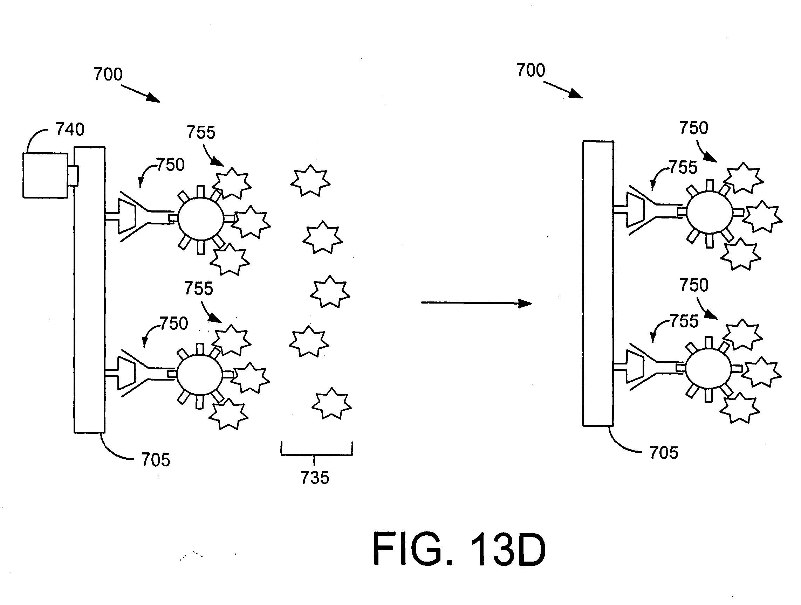

FIG. 13D is a schematic diagram illustrating the removal of the foreign conductive structure 740 from the conductive substrate 705, as well as the separation (*e.g.*, rinsing) of the image nanoparticles 735 and the remaining solution from the image complexes 750 disposed on the conductive substrate 705. Additional preparation and/or treatment of the image complexes 750 can be performed to facilitate their detection.

After sufficient time, the conductive substrate 705 can be analyzed to determine the degree of deposition of the image nanoparticles 755. The biomolecule image of the conductive substrate 705 having image nanoparticles 755 disposed on the target nanoparticles 725 can be obtained using an imaging device and software as described in reference to FIGS. 4A through 4E.

The conditions under which the electrochemical deposition of the image nanoparticles is performed can vary depending upon the design of the biosensor 700. In particular, the following variables can be altered: the type and concentration of the image nanoparticles 735, the type of foreign conductive structure 740, the pH of the solution, the concentration of acid and/or base to achieve the appropriate pH, the temperature of the solution, the type and concentration of the target nanoparticle, the conductive substrate 505, and the type and concentration of the target biomolecule.

### Example 5

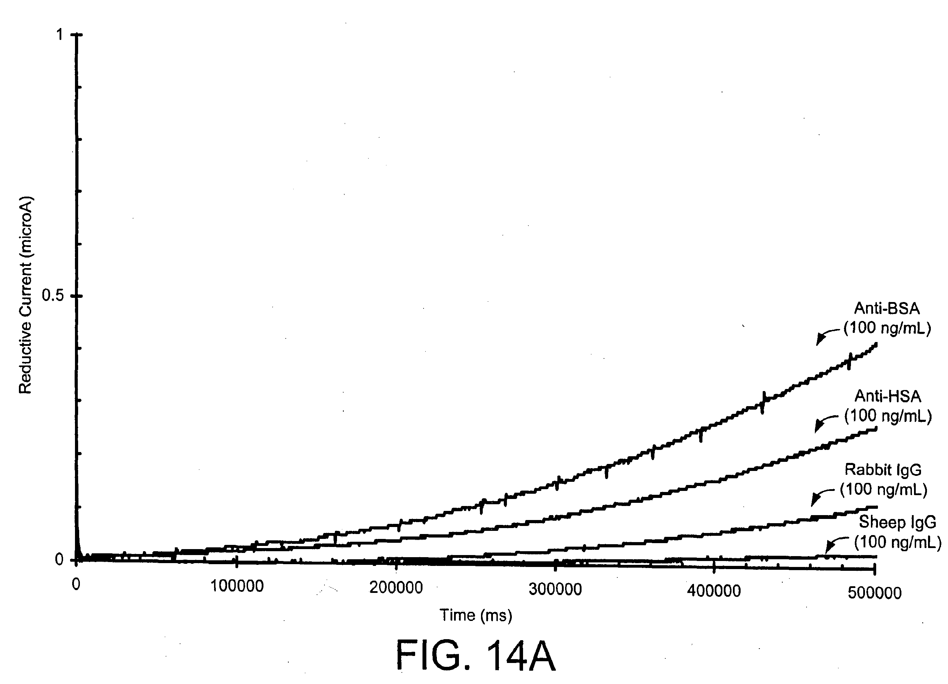

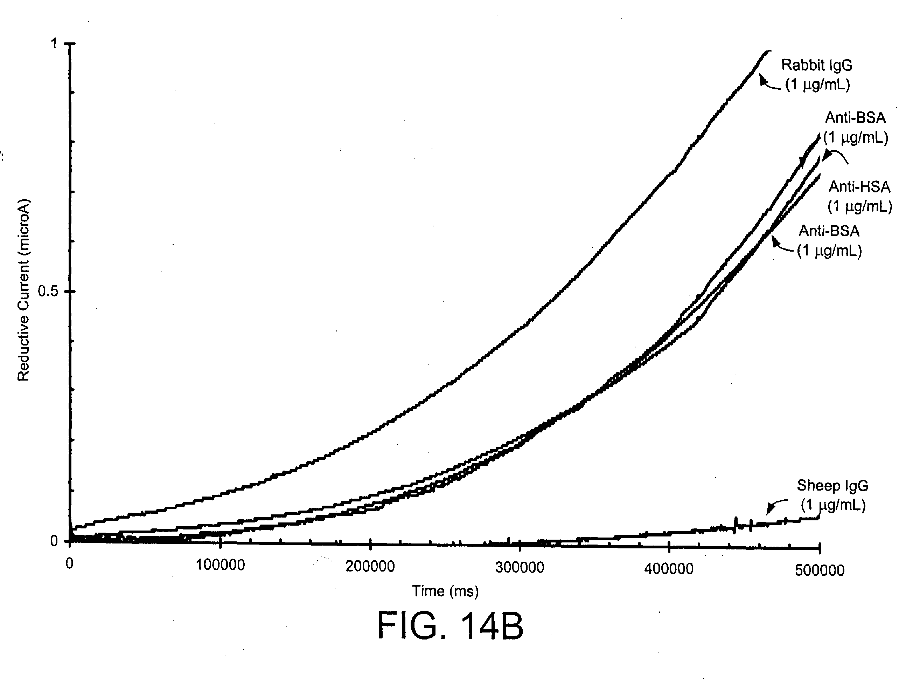

FIGS. 14A and 14B illustrate a process using electrochemical image amplification (*e.g.*, cyclic voltammetry and/or chronoamperometry) to amplify a target biomolecule image for a biosensor system. For this example, the ITO conductive substrates are treated with N-[3-(trimethoxysilyl)propyl]ethylenediamine solution in isopropanol to modify the surface of the conductive substrate to have an affinity for amino groups. The ITO conductive substrates can be purchased from Delta Technologies (Stillwater, MN) and have a resistivity of 8-12 ohms per square inch. Four probe biomolecules, three rabbit IgG (anti-BSA, anti-HSA, and whole molecule rabbit IgG) and one sheep IgG probe biomolecules, are separately made up in solutions with a concentration of about 100 nanogram (ng)/mL (FIG. 14A) and 1 microgram (µg)/mL (FIG. 14B) in a carbonate buffer (3.18 g Na₂CO₃, 5.86 g NaHCO₃, 0.4 g NaN₃ in 200 mL H₂O with pH = 9.6). The probe biomolecules are handspotted onto a patterned ITO conductive substrate. The patterns are circles having about a two millimeter diameter.

Next, the ITO conductive substrate is rinsed with a solution of PBS-Tween buffer (PBST, 0.01M phosphate ion by HNa₂PO₃ and H₂NaPO₃ with pH adjusted to 7.4, with 0.2 g NaN₃, 200 µL Tween-20 in 1 L of deionized water), which can be purchased from Aldrich-Sigma. The ITO conductive substrate is then incubated in a casein blocking solution for 1 h. Then the ITO conductive substrate is rinsed again at least three times with PBST.

A solution of biotinylated anti-rabbit IgG target biomolecules (whole molecule), which is the target biomolecule, with a concentration of about 100 ng/mL (FIG. 14A) and 1µg/mL (FIG. 14B) in ELISA diluent (McCubbin, V. and Frank, M. B., "Detection of Recombinant Proteins by ELISA." *In:* Frank, M. B. ed. *Molecular Biology Protocols.,* 1997, October 2, 1997) iss applied on ITO conductive substrate surface and iss incubated for 2 hours (h) at 37°C in a moist environment. Then, the ITO conductive substrate is rinsed at least three times with PBST.

The ITO conductive substrates are then incubated in a solution of gold-ExtrAvidin conjugate target nanoparticles, which can be purchased from Sigma-Aldrich, for 1h at about 4°C, and subsequently rinsed with PBST.

Electrochemical image amplification is carried out in an aqueous solution containing 2% silver nitrate (AgNO₃) image nanoparticle precursor and 2.5% citric acid. A cyclic voltammogram is performed on the ITO conductive substrate. In this regard, a potential is applied with a CS-1200 potentiostat, which can be purchased from Cypress Systems (Lawrence, KS). The applied potential that is scanned between about a applied potential of -5mV (vs. Ag/AgCl) for up to 10 minutes and change in cathodic current over time was recorded. After the ITO conductive substrate is treated, each ITO conductive substrate is rinsed with deionized water, and dried with nitrogen spray.

FIGS. 14A and 14B show the change in cathodic current for four individual probe biomolecules (three antibodies developed in rabbit (anti-BSA, anti-HSA, and whole molecule rabbit Immunoglobulin G (IgG)) and one sheep IgG) that are deposited on the ITO conductive substrate, as discussed above. In regard to FIG. 14A, the ITO conductive substrate was incubated with a solution having anti-rabbit IgG target biomolecules 100 ng/mL. In regard to the FIG. 14B, the ITO conductive is incubated with a solution having anti-rabbit IgG target biomolecules at a concentration of 1 µg/mL. The anti-rabbit IgG target biomolecules have an affinity for the three rabbit IgG probe biomolecules, but do not have an affinity for the one type of sheep IgG probe biomolecules.

As FIGS. 14A and 14B illustrate, there is significant difference in cathodic currents between the three rabbit-related antibodies (positive control) and one sheep IgG (negative control). The amount of current is proportional to the deposition rate of the silver image nanoparticles, while the amount of charge is proportional to the amount of deposited silver image nanoparticles on each ITO conductive substrate. In FIG. 14B, after the incubation with 1µg/mL positive and negative antibodies, the signal from the positive controls have been amplified for more than 30 times, while keeping positive/negative control ratio at about 20. While the ratio of cathodic currents of the positive/negative controls remains similar, each current is significantly amplified by the disposed silver image nanoparticles, which can enhance signal detection and/or lower the detection limit of the biosensor. Thus, the formation of the silver image nanopartices in FIGS. 14A and 14B illustrates the presence of the anti-rabbit IgG target biomolecules.

### Example 6

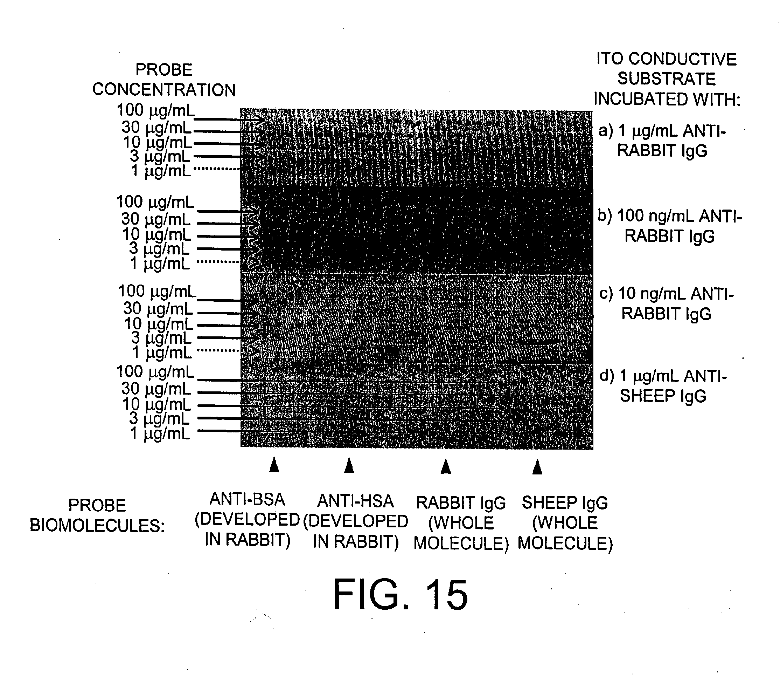

FIG. 15 illustrates a process using electrochemical image amplification to detect a target biomolecule image for a biosensor system. The ITO conductive substrate is similar to the conductive substrate discussed in reference to Example 5. The ITO conductive substrate can purchased from Delta Technologies (Stillwater, MN) and was treated with a solution of poly(L-Lysine (Sigma-Aldrich) to give a positive charge on the substrate. The probe biomolecules: anti-BSA, anti-HSA, whole molecule rabbit IgG, and anti-sheep IgG, are separately made up in solutions with a concentration of about 100 µg/mL, 30 µg/mL, 3 µg/mL, and 1 µg/mL in a carbonate buffer (3.18 g Na₂CO₃, 5.86 g NaHCO₃, 0.4 g NaN₃ in 200 mL H₂O with pH = 9.6).

A handheld arrayer system is used to spot the resultant solutions of the probe biomolecules onto the surface of the ITO conductive substrate. A typical arrayer can be purchased from Schleicher & Schuell Bioscience (Keene, NH). Subsequently, the ITO conductive substrate is incubated in a moist chamber overnight at 4°C.

Next, the ITO conductive substrate is rinsed with a solution of PBS-Tween buffer (PBST, 0.01M phosphate ion by HNa₂PO₃ and H₂NaPO₃ with pH adjusted to 7.4, with 0.2 g NaN₃, 200 µL Tween-20 in 1 L of deionized water), which can be purchased from Sigma-Aldrich. The ITO conductive substrate is then incubated in a casein blocking solution for one h. Then the ITO conductive substrate is rinsed again at least three times with PBST.

The ITO conductive substrate is then incubated in a solution of gold-ExtrAvidin conjugate target nanoparticles, which can be purchased from Sigma-Aldrich, for one h at about 4°C, and subsequently rinsed with PBST.

A galvanostatic silver development is carried out in an aqueous solution containing 2% AgNO₃ image nanoparticle precursor and 2.5% citric acid. A cyclic voltammogram is performed on the ITO conductive substrate. In this regard, a potential is applied with a CS-1200 potentiostat, which can be purchased from Cypress Systems (Lawrence, KS). The applied potential that is scanned between about an applied potential of -5mV (vs. Ag/AgCl) for up to 10 minutes and any change in cathodic current over time was recorded. After the ITO conductive substrate is treated, each ITO conductive substrate is rinsed with deionized water, and dried with nitrogen spray.

FIG. 15 shows the image of a developed antibody array having four probe biomolecules disposed on the ITO conductive substrate. As indicated above, the probe biomolecules include anti-BSA, anti-HSA, and whole molecule rabbit IgG, and anti-sheep IgG. The ITO conductive substrate is then incubated with three biotinylated anti-rabbit IgG (a) 1 µg/mL, b) 100 ng/mL, and c) 10 ng/mL) and one anti-sheep IgG target biomolecules (d)1 µg/mL). The anti-rabbit IgG has an affinity for the anti-BSA, anti-HSA, and whole molecule rabbit IgG, while the anti-sheep has an affinity for anti-sheep IgG. Then the ITO conductive substrate was introduced to a solution having gold-ExtrAvidin target nanoparticles, which have an affinity for the anti-rabbit IgG.

With some variation in spot development, FIG. 15 shows that the anti-rabbit IgG binds to rabbit-related antibody spots. When the anti-sheep IgG is used as the target biomolecule, it binds to sheep IgG spots and creates reversed image. When anti-rabbit IgG is diluted to 10 ng/mL concentration, the differentiation between positive and negative controls becomes unclear compared to the slide incubated with 1 µg/mL anti-rabbit IgG. When 100 ng/mL anti-rabbit IgG is used, the positive and negative controls are clear.

Among the probe biomolecules, the anti-BSA probe biomolecule shows the widest dynamic range with the anti-rabbit IgG target biomolecules (on 1 µg/mL anti-rabbit IgG slide, between 3 µg/mL and 100 µg/mL probe concentration) than other probe biomolecules (only 30 µg/mL and 100 µg/mL spots were developed). However, the intensity of the spots when the probe biomolecule is anti-BSA is not as strong as those having anti-HSA or rabbit IgG probe biomolecules. The same trend can be found on FIGS. 14A and 14B, where anti-BSA probe biomolecule shows the highest cathodic current with 100 ng/mL anti-rabbit IgG incubation, but not the highest with 1 µg/mL anti-rabbit IgG incubation.

The array developed with anti-sheep IgG as target showed similar variation in spot intensity as rabbit IgG with anti-rabbit IgG as target. Only 30 µg/mL and 100 µg/mL probe spots are developed later with silver image nanoparticles.

It should be emphasized that the above-described embodiments are merely possible examples of implementations. Many variations and modifications may be made to the above-described embodiments. All such modifications and variations are intended to be included herein within the scope of this disclosure and protected by the following claims.

## Claims

1. A method for determining the presence of biomolecules, comprising:
providing a first target biomolecule 115, a first target nanoparticle 125, and a first image nanoparticle 135;
forming a first image complex 150 electrochemically on a conductive substrate 105, wherein the first image complex 150 includes the first target biomolecule 115, the first target nanoparticle 125, and the first image nanoparticle 135, wherein the first image nanoparticle 135 is disposed on the first target nanoparticle 125, wherein the first target nanoparticle 125 is disposed on the first target biomolecule 115, and wherein the first target biomolecule 115 is disposed on the conductive substrate 105; and
detecting the first image complex 150, which corresponds to the first target biomolecule 115.

2. The method of claim 1, wherein detecting the first image complex 150 comprises:
detecting the first image complex 150 using an imaging technique.

3. The method of claim 1, wherein detecting the first image complex 150 comprises:
detecting the first image complex 150 using an electronic measurement technique.

4. The method of claim 1, wherein detecting the first image complex 150 comprises:
detecting the first image complex 150 using a mass measurement technique.

5. The method of any preceding claim, wherein the first target nanoparticle 125 includes a gold nanoparticle.

6. The method of any preceding claim, wherein the first image nanoparticle 135 includes a silver nanoparticle.

7. A biosensor system for determining the presence of biomolecules, comprising:
a first target complex 150 disposed on a first conductive substrate 105, wherein the first target complex 150 includes a first target biomolecule 115 and a first target nanoparticle 125, and wherein the first target nanoparticle 125 is disposed on the first target biomolecule 115;
a first image nanoparticle 135 disposed on the first target nanoparticle 125, wherein the first image nanoparticle 135 is electrochemically deposited on the first target nanoparticle 125; and
a detector capable of determining the presence of the first target biomolecule 115 by detecting the first image nanoparticle 135.

8. The system of claim 7, wherein the first target nanoparticle 125 includes a gold nanoparticle.

9. The system of claim 7 or 8, wherein the first image nanoparticle 135 includes a silver nanoparticle.

10. The system of any of claims 7 to 9, wherein the detector is selected from am imaging technique, an electronic measurement technique, and a mass measurement technique.
